# EUROPEAN PATENT SPECIFICATION

(11) **EP 3 363 309 B1**
(45) Date of publication and mention of the grant of the patent: **03.03.2021**
(21) Application number: 18173783.4
(22) Date of filing: 23.05.2018
(51) Int. Cl.: A24F 47/00, A61M 15/06, A61M 11/04

(54) **ATOMIZER AND ELECTRONIC CIGARETTE**
ZERSTÄUBER UND ELEKTRONISCHE ZIGARETTE
DISPOSITIF D'ATOMISATION ET CIGARETTE ÉLECTRONIQUE

(30) Priority: 26.05.2017 CN 201720601546 U
(43) Date of publication of application: 22.08.2018
(73) Proprietor: Shenzhen First Union Technology Co., Ltd., Shenzhen, Guangdong 518103 (CN)
(72) Inventor: LI, Yonghai, Shenzhen, Guangdong 518103 (CN); XU, Zhongli, Shenzhen, Guangdong 518103 (CN); SONG, Hongtao, Shenzhen, Guangdong 518103 (CN)
(74) Representative: Proi World Intellectual Property GmbH

(56) References cited:
- WO-A1-2016/063180
- WO-A1-2016/147188
- WO-A1-2017/011866
- CN-U- 203 555 161
- US-A1- 2017 064 997

## Description

### TECHNICAL FIELD

The present disclosure relates to the technical field of electronic cigarettes, and particularly, to an atomizer having an antifouling structure, and an electronic cigarette having the same atomizer.

### BACKGROUND

As people raise concern about health, they become aware of the harms caused by tobaccos and traditional cigarettes to their bodies. Therefore, electronic cigarette products using tobacco liquids become increasingly applauded by consumers. At present, the electronic cigarettes using tobacco liquids mainly employ the following scheme that: an atomizing unit heats and aerosolizes the tobacco liquid into an aerosol, and the aerosol is expelled via an aerosol channel for a user to inhale. The electronic cigarettes can simulate the effect of smoking traditional cigarettes.

However, the electronic cigarettes using tobacco liquids in the prior art have the following defects. Since the aerosol channel has certain travel, during the smoking process of the electronic cigarettes and the storage process after the smoking, the aerosol cools and is easy to produce condensed droplets, and the condensed droplets are accumulated on an inner wall of the aerosol channel, especially on the position near the mouthpiece. When the user smokes next time, these accumulated condensed droplets are easy to be inhaled to the mouth of the user, or a taste of fried oil is generated. Thus, user experience is impacted.

WO 2016/147188 discloses a vaporizer for vaporizing an active ingredient; US 2017/064997 discloses an electronic vaporizer system; WO 2017/011866 discloses an inhaler device for inhalable liquids; CN 203555161U discloses an atomizer and an electronic cigarette having same; WO 2016/063180 discloses a hydrophobic wrapper.

### SUMMARY

The present invention provides an atomizer according to independent claim 1. The present invention also provides an electronic cigarette comprising such an atomizer according to claim 7. Various improvements are recited in the dependent claims.

The technical problem to be solved by the present disclosure is to overcome the drawbacks in the prior art and provide an atomizer having an antifouling structure and an electronic cigarette having the same atomizer. The antifouling structure is capable of preventing condensed droplets accumulating and adhering to an inner wall of an aerosol channel, thereby avoiding users inhaling the condensed droplets into their mouths when smoking next time.

In order to solve the above technical problem, the present disclosure provides an atomizer having an antifouling structure. The atomizer includes a device housing and an atomizing unit disposed inside the device housing. The device housing defines therein a liquid storage chamber configured for storing tobacco liquid. The atomizing unit is configured for atomizing the tobacco liquid to produce an aerosol that can be inhaled by a user. The device housing further includes an aerosol channel which is in communication with the atomizing unit and is configured for expelling the aerosol. The aerosol channel is formed by a member that has a hydrophobic and oleophobic surface or oleophobic surface disposed on an inner wall thereof.

Specifically, the atomizer further includes an air pipe having an inner chamber and a mouthpiece having an inner chamber, the inner chambers being in communication with each other, the inner chamber of the air pipe and the inner chamber of the mouthpiece together form the aerosol channel, and the hydrophobic and oleophobic surface or oleophobic surface is formed on an inner wall of the air pipe and/or mouthpiece.

In one embodiment, the air pipe or mouthpiece includes a substrate and a fluoropolymer layer or fluorosilicone hydrophobic and oleophobic layer adhered onto an inner wall of the substrate.

In another embodiment, the air pipe or mouthpiece includes a substrate and a nano-material coating adhered onto an inner wall of the substrate, and the nano-material coating is a nano-titanium dioxide layer, a nano-polytetrafluoroethylene layer, a nano-fluorocarbon polymer layer or a nano-siloxane oligomer layer.

In another embodiment, the air pipe is made from a nano-oleophobic material, and the nano-oleophobic material is one or more selected from a group consisting of nano-polytetrafluoroethylene, nano-fluorocarbon polymer and nano-siloxane oligomer.

According to the invention the hydrophobic and oleophobic surface or oleophobic surface has thereon a nano-cantilever structure formed by a plurality of concaves or convexes. Further, the hydrophobic and oleophobic surface or oleophobic surface has a contact angle greater than 150 degrees with respect to a propylene glycol solution or glycerin solution, and has a rolling angle less than 20 degrees with respect to a propylene glycol solution or glycerin solution.

Further, the liquid storage chamber is formed by a component; the component has a hydrophobic and oleophobic surface or a oleophobic surface on an inner wall of the component.

The present disclosure further provides another atomizer having an antifouling structure. The atomizer includes a device housing and an atomizing unit disposed inside the device housing. The device housing defines therein a liquid storage chamber configured for storing tobacco liquid. The atomizing unit is configured for atomizing the tobacco liquid to produce an aerosol that can be inhaled by a user. The liquid storage chamber is formed by a member that has a hydrophobic and oleophobic surface or oleophobic surface disposed on an inner wall thereof.

The present disclosure further provides another atomizer having an antifouling structure. The atomizer includes a device housing and an atomizing unit disposed inside the device housing. The device housing defines therein a liquid storage chamber configured for storing tobacco liquid. The atomizing unit is configured for atomizing the tobacco liquid to produce an aerosol that can be inhaled by a user. The device housing has an outer wall at least partially provided with a hydrophobic and oleophobic surface or oleophobic surface.

The present disclosure further provides an electronic cigarette. The electronic cigarette includes a battery assembly and the atomizer involved in each of the above optimal schemes.

The present disclosure has the following beneficial effects. According to the present disclosure, the aerosol channel of current atomizers is optimized, that is, components which are easy to collect condensed droplets, such as air pipe and mouthpiece, have inner walls coated with oleophobic layers such as nano-material layers or fluoropolymer layers, so as to form a hydrophobic and oleophobic surface or oleophobic surface, so that the condensed droplets cannot be adhered to the inner walls or accumulated on the inner walls for a long time. Therefore, during the smoking process, the condensed droplets formed on the inner wall of the aerosol channel flow back to the inside of the atomizing unit below along the hydrophobic and oleophobic surface or oleophobic surface and are atomized again. The problem of the accumulation of condensed droplets widely existing in current atomizers is effectively solved.

### BRIEF DESCRIPTION OF THE DRAWINGS

FIG. 1 shows an implementation of an atomizer having an antifouling structure provided in an embodiment.
FIG. 2 is a structure diagram of a nano-material coating adhered to an inner wall of an air pipe provided in an embodiment.
FIG. 3 is a structure diagram of an optimal design according to the invention for a hydrophobic and oleophobic
surface or oleophobic surface provided.
FIG. 4 shows another implementation of an atomizer having an antifouling structure provided in an embodiment.
FIG. 5 is a structure diagram of an electronic cigarette provided in an embodiment.

### DETAILED DESCRIPTION

The embodiments below provide multiple implementations for an atomizer having an antifouling structure. A tobacco liquid is loaded in the atomizer. The antifouling structure can improve the oleophobicity of the areas on the atomizer that probably contact the tobacco liquid, so that the tobacco liquid is difficult to adhere to the surfaces of the areas. Thus, users are prevented from inhaling condensed droplets or the surfaces contacting the tobacco liquid are kept clean.

The tobacco liquid involved in the present disclosure includes a solvent and nicotine or other flavor components such as essence and spice. The solvent may include but not limited to one or the mixture of more than one of the following components: polyol, polyol ester (e.g., glyceryl monoacetate, glycerol diacetate, glyceryl triacetate), aliphatic monohydroxy acid ester, aliphatic dihydroxy acid ester or aliphatic polyhydroxy acid ester (e.g., dimethyl dodecanedioate, dimethyl tetradecanedioate). The tobacco liquid used by the atomizer in the present embodiment is common polyols or mixtures thereof, for example, triethylene glycol and 1,3-butanediol, most preferably, glycerol, propylene glycol or a mixture thereof. The present embodiment mainly designs the oleophobic antifouling structure aiming at a tobacco liquid solution containing the above components. Specific embodiments below are provided to further illustrate the atomizer involved in the present disclosure and the antifouling structure and principle of the electronic cigarette having the same atomizer.

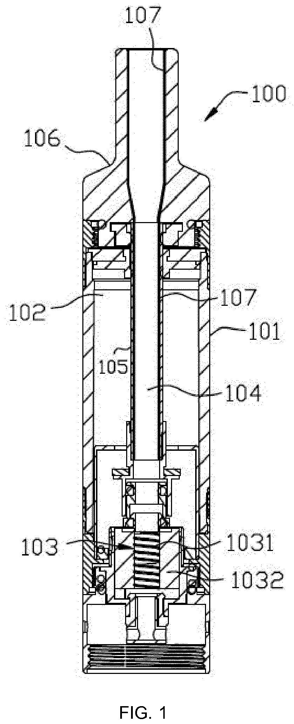

Referring to FIG. 1, the present embodiment provides an atomizer 100 for an electronic cigarette. The atomizer includes a device housing 101 appearing roughly cylindrical and an atomizing unit 103 disposed inside the device housing 101. The device housing 101 defines therein a liquid storage chamber 102 configured for storing tobacco liquid. The atomizing unit 103 is configured for atomizing the tobacco liquid to produce an aerosol that can be inhaled by a user. The device housing 101 further includes an aerosol channel 104 which is in communication with the atomizing unit 103 and is configured for expelling the aerosol. The aerosol channel 104 extends axially along the device housing 101 and has certain travel. The aerosol channel 104 appears a regular sleeve pipe shape. Of course, the aerosol channel 104 can also appear other irregular shapes, for example, S shaped air channel, L shaped air channel, etc.

The atomizing unit 103 has an atomizing chamber therein. The aerosol channel 104 has a lower end connected to the atomizing chamber. The atomizing unit 103 preferably adopts a heating manner, and includes a heating element 1031 and a liquid permeating body 1032 disposed on the periphery of the heating element 1031. The liquid permeating body 1032 guides the tobacco liquid in the liquid storage chamber 102 to the heating element 1031 to heat, so that an aerosol having certain temperature is produced in the aerosol chamber. When a user inhales, the aerosol rises with the air flow and cools and thus is easy to form many condensed droplets on the upper part of the inner side of the aerosol channel 104. As an improvement to the prior art, the aerosol channel 104 is formed by a member that has a hydrophobic and oleophobic surface or oleophobic surface 107 disposed on an inner wall thereof. The condensed droplets (e.g., glycerol and propylene glycol droplets containing nicotine) have a big contact angle on the hydrophobic and oleophobic surface or oleophobic surface 107. Generally, the contact angle is greater than 120 degrees. Therefore, the condensed droplets are difficult to accumulate on the inner wall for a long time, and will flow back to the atomizing chamber to be heated and atomized again. In this way, users are prevented from inhaling the residual tobacco liquid on the inner wall of the member forming the aerosol channel 104 into their mouths when smoking next time.

Specifically, for the member forming the aerosol channel 104, the atomizer 100 further includes an air pipe 105 having an inner chamber and a mouthpiece 106 having an inner chamber, the inner chambers being in communication with each other, the inner chamber of the air pipe 105 and the inner chamber of the mouthpiece 106 together form the aerosol channel 104, and the hydrophobic and oleophobic surface or oleophobic surface 107 is formed on inner walls of the air pipe 105 and the mouthpiece 106, or is formed at least on the inner wall of one of the air pipe 105 and the mouthpiece 106.

The heating element 1031 in the atomizing unit 103 can adopt structures such as heating wire, heating sheet and heating tube. The liquid permeating body 1032 can adopt materials such as cellucotton, non-woven fabric, glass fiber bundle, heat resisting-polymer porous material or microporous ceramic. The heating element in the atomizing unit is a common means in the prior art, and no further description is needed here. Besides the above, in the present embodiment, the atomizing unit 103 can further adopt but not limited to the following: ultrasonic atomizers, air compression atomizers, piezoelectric vibrating atomizers or net type atomizers.

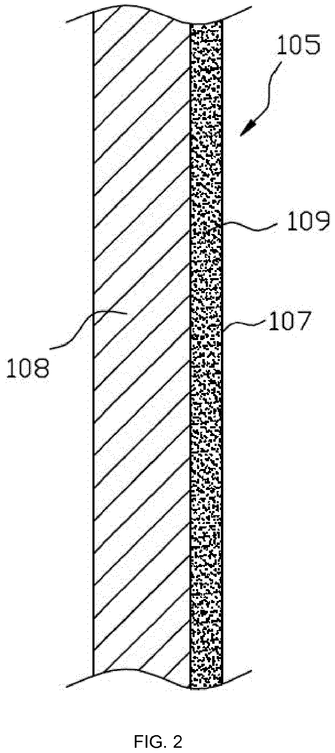

Referring to FIG. 2 and FIG. 1, the hydrophobic and oleophobic surface or oleophobic surface 107 is formed by a nano-material coating adhered to the inner wall of the mouthpiece 106 or air pipe 105. FIG. 2 only shows the coating structure. Herein, the thickness ratio of the substrate 108 to the nano-material coating 109 is not drawn according to the true scale. Taking the air pipe 105 for example, the air pipe 105 includes a substrate 108 and a nano-material coating 109 adhered onto an inner wall of the substrate 108. The nano-material coating 109 has a thickness controlled to be range from 20 micrometers to 30 micrometers. The nano-material coating 109 preferably adopts but not limited to the following materials: a nano-titanium dioxide layer, a nano-polytetrafluoroethylene layer, a nano-fluorocarbon polymer layer or a nano-siloxane oligomer layer. The nano-material coating 109 can be formed by common methods such as sedimentation methods, spray methods, solgel methods, etching methods, etc.

In another embodiment, the air pipe 105 can further include a substrate 108 and a fluoropolymer layer or fluorosilicone hydrophobic and oleophobic layer adhered onto an inner wall of the substrate. The hydrophobicity and/or oleophobicity of the fluorine-containing material are utilized to prevent the condensed droplets soaking the inner wall of the air pipe 105. Specifically, the fluoropolymer layer or fluorosilicone hydrophobic and oleophobic layer can adopt materials such as perfluorooctanoic acid salt oleophobic materials and materials containing perfluoro polyether siloxane, polysiloxane resin or polymethylacrylic acid glycidyl ester.

In another embodiment, the air pipe 105 is a sleeve pipe component made from nano-oleophobic materials. The nano-oleophobic material is one or more selected from a group consisting of nano-polytetrafluoroethylene, nano-fluorocarbon polymer or nano-siloxane oligomer (e.g., polydimethylsiloxane).

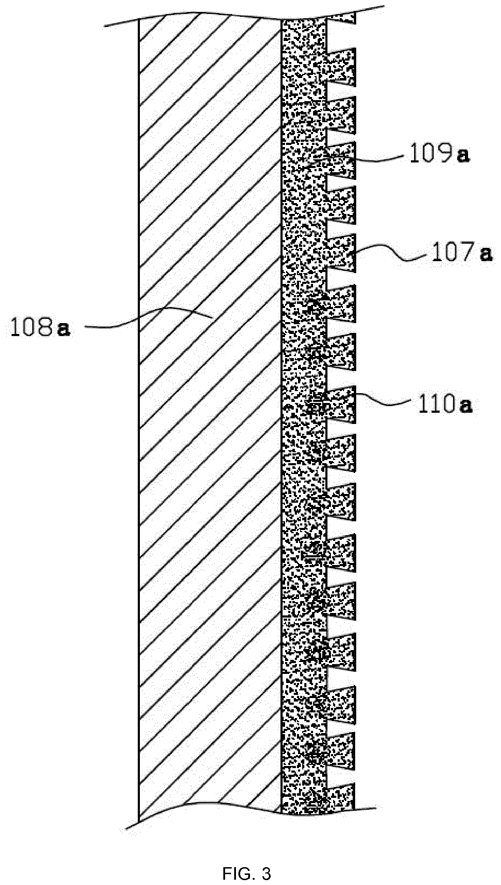

Referring to FIG. 3, as a preferred scheme in the present embodiment, the air pipe 105 can further include a substrate 108a and a nano-material coating 109a. The nano-material coating 109a forms the hydrophobic and oleophobic surface or oleophobic surface 107. According to the invention the hydrophobic and oleophobic surface or oleophobic surface 107a has thereon a nano-cantilever structure 110a formed by a plurality of concaves or convexes. The nano-cantilever structure 110a can further improve the hydrophobicity and/or oleophobicity of the hydrophobic and oleophobic surface or oleophobic surface 107a. Through the nano-cantilever structure 110a, the hydrophobic and oleophobic surface or oleophobic surface 107a has a contact angle greater than 150 degrees with respect to a propylene glycol solution or glycerin solution, and has a rolling angle less than 20 degrees with respect to a propylene glycol solution or glycerin solution. It should be noted that FIG. 3 only shows the schematic diagram of the nano-cantilever structure 110a, rather than the true spacing ratio and height ratio of the convexes or convexes forming the nano-cantilever structure 110a.

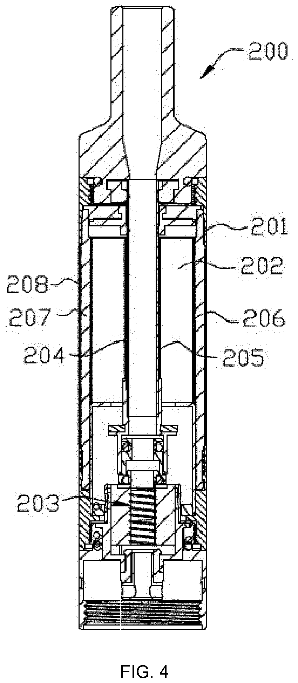

Referring to FIG. 4, the present disclosure further provides an embodiment of another atomizer 200 having an antifouling structure. The atomizer 200 includes a device housing 201 and an atomizing unit 203 disposed inside the device housing 201. The device housing 201 defines therein a liquid storage chamber 202 configured for storing tobacco liquid. The atomizing unit 203 is configured for atomizing the tobacco liquid to produce an aerosol that can be inhaled by a user. The liquid storage chamber 202 is formed by a component that has a hydrophobic and oleophobic surface or oleophobic surface disposed on an inner wall thereof. Specifically, the liquid storage chamber 202 is defined by a circular region spaced between the exterior device housing 201 and an interior air pipe 204. A hydrophobic and oleophobic surface or oleophobic surface 205 is disposed on an outer wall of the air pipe 204. A hydrophobic and oleophobic surface or oleophobic surface 206 is disposed on inner wall of the device housing 201. The coating material can refer to the above description. The hydrophobic and oleophobic surface or oleophobic surfaces 205, 206 can prevent the tobacco liquid adhering to the inner wall of the liquid storage chamber 202, making the inside of the liquid storage chamber 202 convenient to clean. When a user needs to replace the tobacco liquid with a tobacco liquid having other flavors, the original tobacco liquid can be poured out to the maximum extent, thereby preventing tobacco liquids of more than two flavors being mixed to impact the user's taste.

In another preferred design, the device housing 201 has an outer wall at least partially provided with a hydrophobic and oleophobic surface or oleophobic surface 208. For example, the device housing 201 includes a transparent pipe 207, which is made of glass or transparent plastic. The transparent pipe 207 has an outer wall coated with a nano-hydrophobic and oleophobic layer, which has optical transparency and through which a user can observe the tobacco liquid inside the liquid storage chamber 202. The hydrophobic and oleophobic surface or oleophobic surface 208 can prevent overflowing tobacco liquid contaminating the external surface of the device housing 201 when a user refills the tobacco liquid or disassembles the atomizing unit 203. Meanwhile, the hydrophobic and oleophobic surface or oleophobic surface 208 can also prevent too many fingerprints accumulating on the external surface of the device housing 201 and can maintain a good appearance.

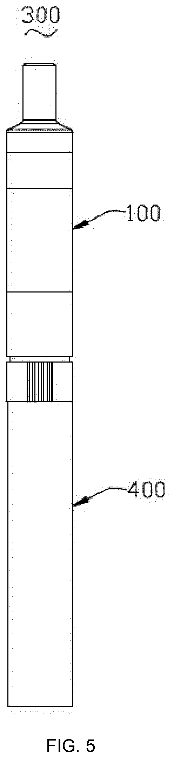

Referring to FIG. 5, the present disclosure provides an embodiment of an electronic cigarette. The electronic cigarette 300 includes a battery assembly 400 and an atomizer 100 connected to the battery assembly 400. The antifouling structure of the atomizer 100 is not described here. Likewise, the above hydrophobic and oleophobic surface or oleophobic surface can also be disposed on an external surface of the battery assembly 400 or a surface of a circuit board inside the battery assembly 400, thereby preventing leaked tobacco liquid contaminating the components.

## Claims

1. An atomizer (100) having an antifouling structure, comprising a device housing (101) and an atomizing unit (103) disposed inside the device housing (101), the device housing (101) defining therein a liquid storage chamber (102) configured for storing tobacco liquid, the atomizing unit (103) being configured for atomizing the tobacco liquid to produce an aerosol, wherein the device housing (101) further comprises an aerosol channel (104) which is in communication with the atomizing unit (103) and is configured for expelling the aerosol, and the aerosol channel (104) is formed by a member that has a hydrophobic and oleophobic surface or oleophobic surface (107) disposed on an inner wall thereof,
**characterized in that** the hydrophobic and oleophobic surface (107) has thereon a nano-cantilever structure formed by a plurality of concaves or convexes.

2. The atomizer (100) according to claim 1, further comprising an air pipe (105) and a mouthpiece (106), the air pipe (105) having an inner chamber, the mouthpiece (106) having an inner chamber, the inner chambers being in communication with each other, wherein the inner chamber of the air pipe (105) and the inner chamber of the mouthpiece (106) together form the aerosol channel (104), and the hydrophobic and oleophobic surface or the oleophobic surface (107) is formed on an inner wall of the air pipe (105) and/or mouthpiece (106).

3. The atomizer (100) according to claim 2, wherein the air pipe (105) or the mouthpiece (106) comprises a substrate (108) and a fluoropolymer layer or fluorosilicone hydrophobic and oleophobic layer adhered onto an inner wall of the substrate.

4. The atomizer (100) according to claim 2, wherein the air pipe (105) or the mouthpiece (106) comprises a substrate (108) and a nano-material coating (109) adhered onto the inner wall of the substrate (108), and the nano-material coating (109) is a nano-titanium dioxide layer, a nano-polytetrafluoroethylene layer, a nano-fluorocarbon polymer layer or a nano-siloxane oligomer layer.

5. The atomizer (100) according to claim 2, wherein the air pipe (105) is made from a nano-oleophobic material, and the nano-oleophobic material is one or more selected from a group consisting of nano-polytetrafluoroethylene, nano-fluorocarbon polymer and nano-siloxane oligomer.

6. The atomizer (100) according to claim 1, wherein the liquid storage chamber (102) is formed by a component, the component has a hydrophobic and oleophobic surface or an oleophobic surface (107) on an inner wall of the component.

7. An electronic cigarette (300), comprising a battery assembly (400) and the atomizer (100) according to any one of claims 1 to 6.

## Patentansprüche

1. Zerstäuber (100) mit einer Antifouling-Struktur, umfassend ein Vorrichtungsgehäuse (101) und eine innerhalb des Vorrichtungsgehäuses (101) angeordnete Zerstäubungseinheit (103), wobei das Vorrichtungsgehäuse (101) darin eine Flüssigkeitsspeicherkammer (102) definiert, die zum Speichern von Tabakflüssigkeit konfiguriert ist, wobei die Zerstäubungseinheit (103) zum Zerstäuben der Tabakflüssigkeit konfiguriert ist, um ein Aerosol zu erzeugen, wobei das Vorrichtungsgehäuse (101) ferner einen Aerosolkanal (104) umfasst, der mit der Zerstäubungseinheit (103) in Verbindung steht und zum Ausstoßen des Aerosols konfiguriert ist, und der Aerosolkanal (104) durch ein Element gebildet ist, das eine hydrophobe und oleophobe Oberfläche oder eine oleophobe Oberfläche (107) aufweist, die an einer Innenwand davon angeordnet ist,
**dadurch gekennzeichnet, dass**
die hydrophobe und oleophobe Oberfläche (107) darauf eine Nano-Cantilever-Struktur aufweist, die durch eine Vielzahl von Konkavitäten oder Konvexen gebildet ist.

2. Zerstäuber (100) nach Anspruch 1, weiterhin umfassend ein Luftrohr (105) und ein Mundstück (106), wobei das Luftrohr (105) eine innere Kammer aufweist, das Mundstück (106) eine innere Kammer aufweist und die inneren Kammern miteinander in Verbindung stehen, wobei die Innenkammer des Luftrohrs (105) und die Innenkammer des Mundstücks (106) zusammen den Aerosolkanal (104) bilden, und die hydrophobe und oleophobe Oberfläche oder die oleophobe Oberfläche (107) an einer Innenwand des Luftrohrs (105) und/oder Mundstücks (106) ausgebildet ist.

3. Zerstäuber (100) nach Anspruch 2, wobei das Luftrohr (105) oder das Mundstück (106) ein Substrat (108) und eine Fluorpolymerschicht oder eine an einer Innenwand des Substrats haftende hydrophobe und oleophobe Fluorsilikonschicht umfasst.

4. Zerstäuber (100) nach Anspruch 2, wobei das Luftrohr (105) oder das Mundstück (106) ein Substrat (108) und eine an der Innenwand des Substrats (108) haftende Nanomaterialbeschichtung (109) umfasst, und die Nanomaterialbeschichtung (109) eine Nano-Titandioxidschicht, eine Nano-Polytetrafluorethylenschicht, eine Nano-Fluorkohlenstoff-Polymerschicht oder eine Nano-Siloxanoligomerschicht ist.

5. Zerstäuber (100) nach Anspruch 2, wobei das Luftrohr (105) aus einem nanooleophoben Material hergestellt ist und das nano-oleophobe Material eines oder mehrere aus der Gruppe bestehend aus Nano-Polytetrafluorethylen, Nano-Fluorkohlenstoff-Polymer und Nano-Siloxanoligomer ist.

6. Zerstäuber (100) nach Anspruch 1, wobei die Flüssigkeitsspeicherkammer (102) durch eine Komponente gebildet ist, wobei die Komponente eine hydrophobe und oleophobe Oberfläche oder eine oleophobe Oberfläche (107) an einer Innenwand der Komponente aufweist.

7. Elektronische Zigarette (300), umfassend eine Batterieanordnung (400) und den Zerstäuber (100) nach einem der Ansprüche 1 bis 6.

## Revendications

1. Pulvérisateur (100) ayant une structure antisalissure, comprenant un logement de dispositif (101) et une unité de pulvérisation (103) disposée à l'intérieur du logement de dispositif (101), le logement de dispositif (101) définissant dans celui-ci une chambre de stockage de liquide (102) configurée pour stocker du liquide de tabac, l'unité de pulvérisation (103) étant configurée pour pulvériser le liquide de tabac pour produire un aérosol, dans lequel le logement du dispositif (101) comprend en outre un canal d'aérosol (104) qui est en communication avec l'unité de pulvérisation (103) et est configuré pour expulser l'aérosol, et le canal d'aérosol (104) est formé par un élément qui a une surface hydrophobe et oléophobe ou une surface oléophobe (107) disposée sur une paroi interne de celui-ci,
**caractérisé en ce que**
la surface hydrophobe et oléophobe (107) présente sur celle-ci une structure de nano-canal de levier formée par une pluralité de concaves ou de convexes.

2. Pulvérisateur (100) selon la revendication 1, comprenant en outre un tuyau d'air (105) et un embout (106), le tuyau d'air (105) ayant une chambre intérieure, l'embout (106) ayant une chambre intérieure, les chambres intérieures étant en communication les unes avec les autres, dans lequel la chambre intérieure du tuyau d'air (105) et la chambre intérieure de l'embout (106) forment ensemble le canal d'aérosol (104), et la surface hydrophobe et oléophobe ou la surface oléophobe (107) est formée sur une paroi intérieure du tuyau d'air (105) et/ou de l'embout (106).

3. Pulvérisateur (100) selon la revendication 2, dans lequel le tuyau d'air (105) ou l'embout (106) comprend un substrat (108) et une couche de fluoropolymère ou une couche hydrophobe et oléophobe de fluorosilicone collée sur une paroi interne du substrat.

4. Pulvérisateur (100) selon la revendication 2, dans lequel le tuyau d'air (105) ou l'embout (106) comprend un substrat (108) et un revêtement de nanomatériau (109) collé sur la paroi interne du substrat (108), et le revêtement de nanomatériau (109) est une couche de nano-dioxyde de titane, une couche de nanopolytétrafluoroéthylène, une couche de polymère de nanofluorocarbone ou une couche d'oligomère de nanosiloxane.

5. Pulvérisateur (100) selon la revendication 2, dans lequel le tuyau d'air (105) est fabriqué à partir d'un matériau nano-oléophobe, et le matériau nano-oléophobe est un ou plusieurs matériaux choisis dans un groupe constitué par le nanopolytétrafluoroéthylène, un polymère de nano-fluorocarbone et un oligomère de nano-siloxane.

6. Pulvérisateur (100) selon la revendication 1, dans lequel la chambre de stockage de liquide (102) est formée par un composant, le composant a une surface hydrophobe et oléophobe ou une surface oléophobe (107) sur une paroi interne du composant.

7. Cigarette électronique (300), comprenant un ensemble de batterie (400) et l'atomiseur (100) selon l'une quelconque des revendications 1 à 6.
